(19) Europäisches Patentamt
European Patent Office
Office européen des brevets

(11) Veröffentlichungsnummer: **0 235 676**
**B1**

(12) **EUROPÄISCHE PATENTSCHRIFT**

(45) Veröffentlichungstag der Patentschrift: **13.06.90**

(21) Anmeldenummer: **87102290.1**

(22) Anmeldetag: **18.02.87**

(51) Int. Cl.$^5$: **C 07 D 401/04,**
C 07 D 471/04, A 61 K 31/47,
A 61 K 31/435 // (C07D471/04,
221:00, 221:00)

(54) **7-(1-Pyrrolidinyl)-chinoloncarbonsäure-Derivate.**

(30) Priorität: **01.03.86 DE 3606698**

(43) Veröffentlichungstag der Anmeldung:
**09.09.87 Patentblatt 87/37**

(45) Bekanntmachung des Hinweises auf die Patenterteilung:
**13.06.90 Patentblatt 90/24**

(84) Benannte Vertragsstaaten:
**AT BE CH DE ES FR GB GR IT LI NL SE**

(56) Entgegenhaltungen:
**EP-A-0 047 005**
**EP-A-0 153 828**
**EP-A-0 160 578**
**US-A-4 571 396**

(73) Patentinhaber: **BAYER AG**
**D-5090 Leverkusen 1 Bayerwerk (DE)**

(72) Erfinder: **Petersen, Uwe, Dr.**
**Auf dem Forst 4**
**D-5090 Leverkusen (DE)**
Erfinder: **Grohe, Klaus, Dr.**
**Am Wasserturm 10**
**D-5068 Odenthal (DE)**
Erfinder: **Zeiler, Hans-Joachim, Dr.**
**Elsbeeker Strasse 46**
**D-5620 Velbert (DE)**
Erfinder: **Metzger, Karl Georg, Dr.**
**Pahlkestrasse 75**
**D-5600 Wuppertal 1 (DE)**

**Die Akte enthält technische Angaben, die nach dem Eingang der Anmeldung eingereicht wurden und die nicht in dieser Patentschrift enthalten sind.**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 235 676 B1

**Beschreibung**

Die vorliegende Erfindung betrifft neue 7-(1-Pyrrolidinyl)-3-chinoloncarbonsäure-Derivate, Verfahren zu ihrer Herstellung sowie diese enthaltende antibakterielle Mittel und Futterzusatzstoffe.

Es ist bereits bekannt geworden, daß 1-Cyclopropyl-6-fluor-1,4-dihydro-4-oxo-7-(1-piperazinyl)-3-chinolincarbonsäure (Ciprofloxacin, DE—OS 3 142 854) und 8-Chlor-1-cyclopropyl-6-fluor-1,4-dihydro-4-oxo-7-(1-piperazinyl)-3-chinoloncarbonsäuren (DE—OS 3 420 734) hochwirksame antibakterielle Mittel sind.

In EP—A—0 153 828, EP—A—0 160 578, EP—A—0 047 005 und US—A—4 571 396 werden ebenfalls Chinolon- bzw. Naphthyridoncarbonsäuren mit antibakterieller Wirkung beschrieben.

Es wurde nun gefunden, daß die neuen 7-(1-Pyrrolidinyl)-3-chinoloncarbonsäure-Derivate der Formel (I)

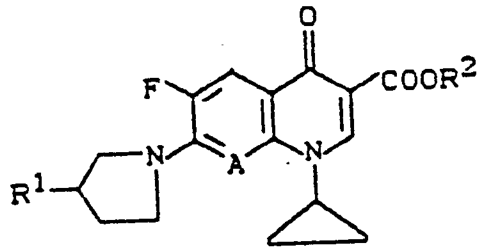

(I),

in welcher

A für CH, CCl oder CF,

$R^1$ für Hydroxy oder Hydroxymethyl und

$R^2$ für Wasserstoff, Alkyl mit 1 bis 4 Kohlenstoffatomen oder (5-Methyl-2-oxo-1,3-dioxol-4-yl)-methyl steht, mit der Maßgabe, daß für den Fall, daß $R^1$ für Hydroxy steht, A nicht für den Rest CF stehen kann, und deren pharmazeutisch verwendbaren Hydrate sowie die Alkali-, Erdalkali-, Silber- und Guanidiniumsalze der zugrundeliegenden Carbonsäuren eine hohe antibakterielle Wirkung im grampositiven und gramnegativen Bereich aufweisen.

Sie eignen sich daher als Wirkstoffe für die Human- und Veterinärmedizin, wobei zur Veterinärmedizin auch die Behandlung von Fischen zur Therapie oder Vorbeugung bakterieller Infektionen zu zählen ist.

Weiterhin wurde gefunden, daß man die Verbindungen der Formel (I) erhält, wenn man Verbindungen der Formel (II)

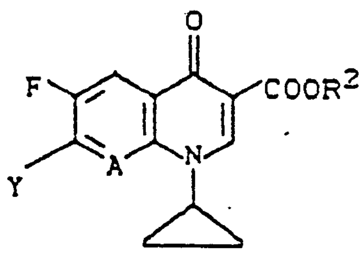

(II),

in welcher

A und $R^2$ die oben angegebene Bedeutung haben und

Y für Halogen, insbesondere Fluor oder Chlor, steht, mit Pyrrolidinen der Formel (III)

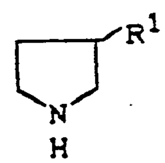

(III),

in welcher $R^1$ die oben angegebene Bedeutung hat, gegebenenfalls in Gegenwart von Säurebindungsmitteln umsetzt (Methode A).

Erfindungsgemäße Verbindungen der Formel (I)

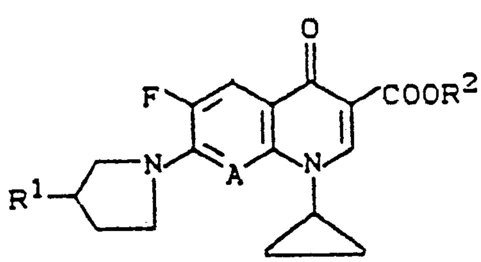

(I),

in welcher A, $R^1$ und $R^2$ die oben angegebene Bedeutung haben, aber $R^2$ nicht Wasserstoff sein kann, können auch erhalten werden, indem man eine Verbindung (IV)

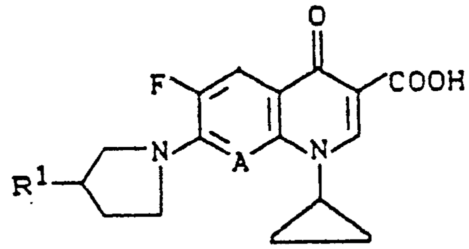

(IV),

in welcher $R^1$ und A die oben angegebene Bedeutung haben, mit Verbindungen der Formel (V)

$$R^2—Z \qquad (V),$$

in welcher $R^2$ die oben angegebene Bedeutung hat, aber nicht Wasserstoff sein kann, und

Z für Hydroxy oder Halogen, insbesondere Chlor, Brom oder Iod, steht, gegebenenfalls in Gegenwart von Säurebindungsmitteln oder unter wasserabspaltenden Bedingungen umsetzt (Methode B).

Verwendet man bei der Umsetzung nach Methode A 1-Cyclopropyl-6,7-difluor-1,4-dihydro-4-oxo-3-chinolincarbonsäure und 3-Hydroxypyrrolidin als Ausgangsstoffe, so kann der Reaktionsablauf durch das folgende Formelschema wiedergegeben werden:

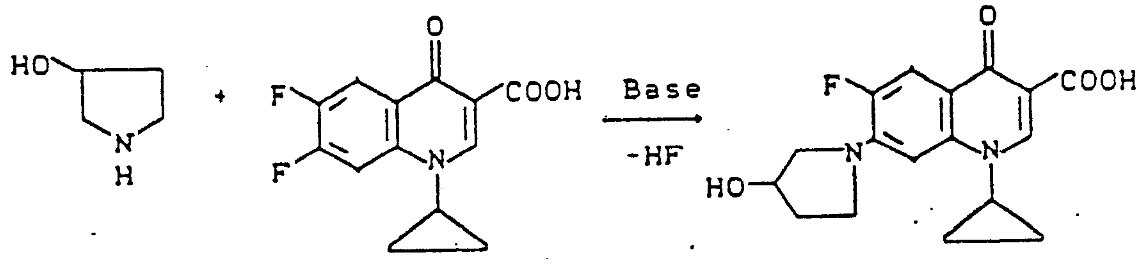


Verwendet man bei der Umsetzung nach Methode B 1-Cyclopropyl-6-fluor-1,4-dihydro-7-(3-hydroxy-1-pyrrolidinyl)-4-oxo-3-chinolincarbonsäure und Ethylakohol als Ausgangsstoffe, so kann der Reaktionsblauf durch das folgende Formelschema wiedergegeben werden:

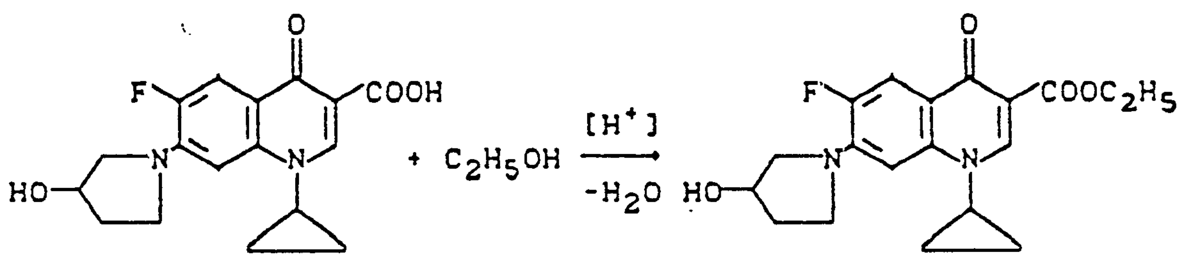


Die als Ausgangsstoffe verwendeten Verbindungen der Formel (II) sind bekannt. Als Beispiele seien genannt:

7 - Chlor - 1 - cyclopropyl - 6 - fluor - 1,4 - dihydro - 4 - oxo - 3 - chinolincarbonsäure (Deutsche Patentanmeldung 3 142 854),

1 - Cyclopropyl - 6,7 - difluor - 1,4 - dihydro - 4 - oxo - 3 - chinolincarbonsäure (Europäische Patentanmeldung 113 091),

8 - Chlor - 1 - cyclopropyl - 6,7 - difluor - 1,4 - dihydro - 4 - oxo - 3 - chinolincarbonsäure (Deutsche Patentanmeldung 3 420 743),

1 - Cyclopropyl - 6,7,8 - trifluor - 1,4 - dihydro - 4 - oxo - 3 - chinolincarbonsäure (Deutsche Patentanmeldung 3 318 145),

7 - Chlor - 1 - cyclopropyl - 6 - fluor - 1,4 - dihydro - 4 - oxo - 1,8 - naphthyrin - 3 - carbonsäure,

7 - Chlor - 1 - cyclopropyl - 6 - fluor - 1,4 - dihydro - 4 - oxo - 3 - chinolincarbonsäureethylester (Deutsche Patentanmeldung 3 142 854),

1 - Cyclopropyl - 6,7 - difluor - 1,4 - dihydro - 4 - oxo - 3 - chinolincarbonsäureethylester (Europäische Patentanmeldung 113 091),

8 - Chlor - 1 - cyclopropyl - 6,7 - difluor - 1,4 - dihydro - 4 - oxo - 3 - chinolincarbonsäureethylester (Deutsche Patentanmeldung 3 420 743),

1 - Cyclopropyl - 6,7,8 - trifluor - 1,4 - dihydro - 4 - oxo - 3 - chinolincarbonsäureethylester (Deutsche Patentanmeldung 3 318 145).

Die als Ausgangsverbindungen verwendeten Pyrrolidine der Formel (III) sind bekannt. Die Verbindungen können sowohl als Racemate als auch als reine Enantiomere, die nach gängigen Methoden erhalten werden können, eingesetzt werden. Folgende Verbindungen können eingesetzt werden:

(±)-3-Hydroxy-pyrrolidin,
(+)-3-Hydroxy-pyrrolidin,
(−)-3-Hydroxy-pyrrolidin,
(±)-3-Hydroxymethyl-pyrrolidin,
(+)-3-Hydroxymethyl-pyrrolidin,
(−)-3-Hydroxymethyl-pyrrolidin.

Die als Ausgangsverbindungen für die Methode B verwendeten Verbindungen der Formel (IV) sind neu und werden nach der Methode A des erfindungsgemäßen Verfahrens hergestellt.

Die als Ausgangsverbindungen verwendeten Verbindungen der Formel (V) sind bekannt. Als Beispiele seien genannt: Methanol, Ethanol, Propanol, Isopropanol, Butanol, Isobutanol, sek.-Butanol, tert.-Butanol, Methyliodid, Ethylbromid, Propylchlorid, 4-Brom-methyl- oder 4-Chlormethyl-5-methyl-1,3-dioxol-2-on.

Die Umsetzung von (II) mit (III) gemäß Methode A, bei der die Pyrrolidine (III) auch in Form ihrer Salze, wie z.B. der Hydrochloride, Hydrobromide, Sulfate oder Acetate, eingesetzt werden können, wird vorzugsweise in einem Verdünnungsmittel wie Dimethylsulfoxide, N,N-Dimethylformamid, Hexamethylphosphorsäuretrisamid, Sulfolan, Acetonitril, Wasser, einem Alkohol wie Methanol, Ethanol, n-Propanol, Isopropanol, Glykolmonomethylether oder Pyridin vorgenommen. Ebenso können Gemische dieser Verdünnungsmittel verwendet werden.

Als Säurebinder können alle üblichen anorganischen und organischen Säurebindungsmittel verwendet werden. Hierzu gehören vorzugsweise die Alkalihydroxide, Alkalicarbonate, organische Amine und Amidine. Als besonders geeignet seien im einzelnen genannt: Triethylamin, 1,4-Diaza-bicyclo-[2.2.2]octan (DABCO), 1,8-Diazabicyclo[5,4,0]undec-7-en (DBU) oder überschüssiges Pyrrolidin (III).

Die Reaktionstemperaturen können in einem größeren Bereich variiert werden. Im allgemeinen arbeitet man zwischen etwa 20 und 200°C, vorzugsweise zwischen 80 und 180°C.

Die Umsetzung kann bei Normaldruck, aber auch bei erhöhtem Druck durchgeführt werden. Im allgemeinen arbeitet man bei Drucken zwischen etwa 1 und 100 bar, vorzugsweise zwischen 1 und 10 bar.

Bei der Durchführung des erfindungsgemäßen Verfahrens setzt man auf 1 Mol der Carbonsäure (II) 1 bis 15 Mol, vorzugsweise 1 bis 6 Mol der Verbindung (III) ein.

Freie Hydroxygruppen können während der Umsetzung durch eine geeignete Hydroxyschutzgruppe, zum Beispiel durch den Tetrahydropyranylrest, geschützt und nach Beendigung der Reaktion wieder freigesetzt werden.

Zur Herstellung der erfindungsgemäßen Ester gemäß Methode B wird die zugrundeliegende Carbonsäure der Formel (IV) vorzugsweise in überschüssigem Alkohol in Gegenwart von starken Säuren, wie Schwefelsäure, wasserfreiem Chlorwasserstoff, Methansulfonsäure, p-Toluolsulfonsäure oder sauren Ionenaustauschern, bei Temperaturen von etwa 20° bis 200°C, vorzugsweise etwa 60° bis 120°C umgesetzt. Das entstehende Reaktionswasser kann auch durch azeotrope Destillation mit Chloroform, Tetrachlormethan, Benzol oder Toluol entfernt werden.

Man kann die Alkylester ebenfalls durch Umsetzung eines Alkali- oder Aminsalzes der zugrundeliegenden Carbonsäure der Formel (IV) mit einem Alkylhalogenid vorzugsweise in einem Verdünnungsmittel wie Dimethylsulfoxid, Dimethylformamid, Pyridin, Sulfolan oder Tetramethylharnstoff bei Temperaturen von etwa 0°C bis etwa 150°C, vorzugsweise bei 10°C bis 100°C, erhalten.

Die als Prodrug verwendeten (5-Methyl-2-oxo-1,3-dioxol-4-yl-methyl)-ester werden durch Umsetzung eines Alkalisalzes der zugrundeliegenden Carbonsäure mit 4-Brommethyl- oder 4-Chlormethyl-5-methyl-1,3-dioxol-2-on in einem Lösungsmittel wie Dimethylformamid, Dimethylacetamid, Dimethylsulfoxid oder Tetramethylharnstoff bei Temperaturen von etwa 0°C bis 100°C, vorzugsweise 0°C bis 50°C, erhalten.

Die Alkali- oder Erdalkalisalze der erfindungsgemäßen Carbonsäuren werden beispielsweise durch Lösen der Säure in unterschüssiger Alkali- oder Erdalkalilauge, Filtration und Eindampfen des Filtrats bis zur Trockne, erhalten. Pharmazeutisch geeignet sind Natrium-, Kalium- oder Calciumsalze. Durch Umsetzung eines Alkali- oder Erdalkalisalzes mit einem geeigneten Silbersalz wie Silbernitrat werden die entsprechenden Silbersalze erhalten.

Die erfindungsgemäßen Wirkstoffe können sowohl als Racemate als auch als enantiomerenreine Verbindungen vorliegen.

Außer den in den Beispielen aufgeführten Verbindungen seien als neue Wirkstoffe im einzelnen genannt:

1-Cyclopropyl-6-fluor-1,4-dihydro-7-[3-(S)-hydroxy-1-pyrrolidinyl]-4-oxo-3-chinolincarbonsäure, 1-Cyclopropyl-6-fluor-1,4-dihydro-7-[3-(R)-hydroxy-1-pyrrolidinyl]-4-oxo-3-chinolincarbonsäure, 8-Chlor-1-cyclopropyl-6-fluor-1,4-dihydro-7-[3-(S)-hydroxy-1-pyrrolidinyl]-4-oxo-3-chinolincarbonsäure, 8-Chlor-1-cyclopropyl-6-fluor-1,4-dihydro-7-[3-(R)-hydroxy-1-pyrrolidinyl]-4-oxo-3-chinolincarbonsäure, Natrium-1-cyclopropyl-6-fluor-1,4-dihydro-7-[3-(S)-hydroxy-1-pyrrolidinyl]-4-oxo-3-chinolincarboxylat, Kalium-1-cyclopropyl-6-fluor-1,4-dihydro-7-[3-(R)-hydroxy-1-pyrrolidinyl]-4-oxo-3-chinolincarboxylat, Natrium-8-chlor-1-cyclopropyl-6-fluor-1,4-dihydro-7-[3-(S)-hydroxy-1-pyrrolidinyl]-4-oxo-3-chinolincarboxylat, Silber-8-chlor-1-cyclopropyl-6-fluor-1,4-dihydro-7-[3-(R)-hydroxy-1-pyrrolidinyl]-4-oxo-3-chinolincarboxylat, 1-Cyclopropyl-6-fluor-1,4-dihydro-7-[3-(S)-hydroxy-1-pyrrolidinyl]-4-oxo-3-chinolincarbonsäure-methylester, 1-Cyclopropyl-6-fluor-1,4-dihydro-7-[3-(R)-hydroxy-1-pyrrolidinyl]-4-oxo-3-chinolincarbonsäure-ethylester, 8-Chlor-1-cyclopropyl-6-fluor-1,4-dihydro-7-[3-(S)-hydroxy-1-pyrrolidinyl]-4-oxo-3-chinolincarbonsäure-ethylester, 8-Chlor-1-cyclopropyl-6-fluor-1,4-dihydro-7-[3-(R)-hydroxy-1-pyrrolidinyl]-4-oxo-3-chinolincarbonsäure-propylester, 1-Cyclopropyl-6-fluor-1,4-dihydro-7-[3-(S)-hydroxy-1-pyrrolidinyl]-4-oxo-3-chinolincarbonsäure-(5-methyl-2-oxo-1,3-dioxol-4-yl-methyl)-ester, 1-Cyclopropyl-6-fluor-1,4-dihydro-7-[3-(R)-hydroxy-1-pyrrolidinyl]-4-oxo-3-chinolincarbonsäure-(5-methyl-2-oxo-1,3-dioxol-4-yl-methyl)-ester, 8-Chlor-1-cyclopropyl-6-fluor-1,4-dihydro-7-[3-(S)-hydroxy-1-pyrrolidinyl]-4-oxo-3-chinolincarbonsäure-(5-methyl-2-oxo-1,3-dioxol-4-yl-methyl)-ester, 8-Chlor-1-cyclopropyl-6-fluor-1,4-dihydro-7-[3-(R)-hydroxy-1-pyrrolidinyl]-4-oxo-3-chinolincarbonsäure-(5-methyl-2-oxo-1,3-dioxol-4-yl-methyl)-ester, 1-Cyclopropyl-6-fluor-1,4-dihydro-7-[3-(S)-hydroxymethyl-1-pyrrolidinyl]-4-oxo-3-chinolincarbonsäure, 1-Cyclopropyl-6-fluor-1,4-dihydro-7-[3-(R)-hydroxymethyl-1-pyrrolidinyl]-4-oxo-3-chinolincarbonsäure, 8-Chlor-1-cyclopropyl-6-fluor-1,4-dihydro-7-[3-(S)-hydroxymethyl-1-pyrrolidinyl]-4-oxo-3-chinolincarbonsäure, 8-Chlor-1-cyclopropyl-6-fluor-1,4-dihydro-7-[3-(R)-hydroxymethyl-1-pyrrolidinyl]-4-oxo-3-chinolincarbonsäure, 1-Cyclopropyl-6,8-difluor-1,4-dihydro-7-(3-hydroxymethyl-1-pyrrolidinyl]-4-oxo-3-chinolincarbonsäureethylester.

Beispiel für eine erfindungsgemäße Tablette

Jede Tablette enthält:

| | |
|---|---|
| Verbindung des Beispiels 1 | 583,0 mg |
| Mikrokristalline Cellulose | 55,0 mg |
| Maisstärke | 72,0 mg |
| Poly-(1-vinyl-2-pyrrolidon) unlöslich | 30,0 mg |
| Hochdisperses Siliciumdioxid | 5,0 mg |
| Magnesiumstearat | 5,0 mg |
| | 750,0 mg |

Die Lackhülle enthält:

| | |
|---|---|
| Poly-(O-hydroxypropyl-O-methyl)-cellulose 15 cp | 6,0 mg |
| Marcrogol 4000 rec. INN Polyethylenglykol (DAB) | 2,0 mg |
| Titan-(IV)-oxid | 2,0 mg |
| | 10,0 mg |

Die erfindungsgemäßen Verbindungen zeigen bei geringer Toxizität ein breites antibakterielles Spektrum gegen gram-positive un gram-negative Keime, insbesondere gegen Enterobakteriaceen; vor allem auch gegen solche, die resistent sind gegen verschiedene Antibiotika, wie z.B. Penicilline, Cephalosporine, Aminoglykoside, Sulfonamide, Tetracycline.

Diese wertvollen Eigenschaften ermöglichen ihre Verwendung als chemotherapeutische Wirkstoffe in der Medizin sowie als Stoffe zur Konservierung von anorganischen und organischen Materialien, insbesondere von organischen Materialien aller Art, z.B. Polymeren, Schmiermitteln, Farben, Fasern, Leder, Papier und Holz, von Lebensmitteln und von Wasser.

Die erfindungsgemäßen Verbindungen sind gegen ein sehr breites Spektrum von Mikroorganismen wirksam. Mit ihrer Hilfe können gram-negative und gram-positive Bakterien und bakterienänliche Mikroorganismen bekämpft sowie die durch diese Erreger hervorgerufenen Erkrankungen verhindert, gebessert und/oder geheilt werden.

Besonders wirksam sind die erfindungsgemäßen Verbindungen gegen Bakterien und bakterienähnliche Mikroorganismen. Sie sind daher besonders gut zur Prophylaxe und Chemotherapie von lokalen und systemischen Infektionen in der Human- und Tiermedizin geeignet, die durch diese Erreger hervorgerufen werden.

Beispielsweise können lokale und/oder systemische Erkrankungen behandelt und/oder verhindert werden, die durch die folgenden Erreger oder durch Mischungen der folgenden Erreger verursacht werden: Gram-positive Kokken, z.B. Staphylokokken (Staph. aureus, Staph. epidermidis) und Streptokokken (Strept, agalactiae, Strept, faecalis, Strept. pneumoniae, Strept. pyogenes); gram-negative Kokken (Neisseria gonorrhoeae) sowie gram-negative Stäbchen wie Enterobakteriaceen, z.B. Escherichia coli, Haemophilus influenzae, Citrobacter (Citrob. freundii, Citrob. divernis), Salmonella und Shigella; ferner Klebsiellen (Klebs. pneumoniae, Klebs. oxytoca), Enterobacter (Ent. aerogenes, Ent. agglomerans), Hafnia, Serratia (Serr. marcescens), Proteus (Pr. mirabilis, Pr. rettgeri, Pr. vulgaris), Providencia, Yersinia, sowie die Gattung Acinetobacter. Darüber hinaus umfaßt das antibakterielle Spektrum die Gattung Pseudomonas (Ps. aeruginosa, Ps. maltophilia) sowie strikt anaerobe Bakterien wie z.B. Bacteroides fragilis, Vertreter der Gattung Peptococcus, Peptostreptococcus sowie die Gattung Clostridium; ferner Mykoplasmen (M. pneumoniae, M. hominis. M. urealyticum) sowie Mykobakterien, z.B. Mycobacterium tuberculosis.

Die obige Aufzählung von Erregern ist lediglich beispielhaft und keineswegs beschränkend aufzufassen. Als Krankheiten, die durch die genannten Erreger oder Mischinfektionen verursacht und durch die erfindungsgemäßen Verbindungen verhindert, gebessert oer geheilt werden können, seien beispielsweise genannt:

Infektionskrankheiten beim Menschen wie zum Beispiel Otitis, Pharyngitis, Pneumonie, Peritonitis, Pyelonephritis, Cystitis, Endocarditis, Systeminfektionen, Bronchitis (akut, chronisch), septische Infektionen, Erkrankungen der oberen Luftwege, diffuse Panbronchiolitis, pulmonäres Emphysem, Dysenterie, Enteritis, Leberabszesse, Urethritis, Prostatitis, Epididymitis, gastrointerstinale Infektionen, Knochen- und Gelenkinfektionen, zystische Fibrose, Hautinfektionen, postoperative Wundinfektionen, Abszesse, Phlegmone, Wundinfektionen, infizierte Verbrennungen, Brandwunden, Infektionen im Mundbereich, Infektionen nach Zahnoperationen, Osteomyelitis, septische Arthritis, Cholecystitis, Peritonitis mit Appendicitis, Cholangitis, intraabdominale Abszesse, Pankreatitis, Sinusitis, Mastoiditis, Mastitis, Tonsillitis, Typhus, Meningitis und Infektionen des Nervensystems, Salpingitis, Endometritis, Genital-Infektionen, Pelveoperitonitis und Augeninfektionen.

Außer beim Menschem können bakterielle Infektionen auch bei anderen Spezies behandelt werden. Beispielhaft seien genannt:

Schwein: Coli-diarrhoe, Enterotoxämie, Sepsis, Dysenterie, Salmonellose, Mastitis-Metritis-Agalaktie-Syndrom, Mastitis;

Wiederkäuer (Rind, Schaf, Ziege): Diarrhoe, Sepsis, Bronchopneumonie, Salmonellose, Pasteurellose, Mykoplasmose, Genitalinfektionen;

Pferd: Bronchopneumonien, Fohlenlähme, puerperale und postpuerperale Infektionen, Salmonellose;

Hund und Katze: Bronchopneumonie, Diarrhoe, Dermatitis, Otitis, Harnwegsinfekte, Prostatitis;

Geflügel (Huhn, Pute, Wachtel, Taube, Ziervögel und andere): Mycoplasmose, E. coli-Infektionen, chronische Luftwegserkrankungen, Salmonellose, Pasteurellose, Psittakose.

Ebenso können bakterielle Erkrankungen bei der Aufzucht und Haltung von Nutz- und Zierfischen behandelt werden, wobei sich das antibakterielle Spektrum über die vorher genannten Erreger hinaus auf weitere Erreger wie zum Beispiel Pasteurella, Brucella, Campylobacter, Listeria, Erysipelothrix, Corynebakterien, Borrelia, Treponema, Nocardia, Rikettsien, Yersinia, erweitert.

Zur vorliegenden Erfindung gehören pharmazeutische Zubereitungen, die neben nicht-toxischen, inerten pharmazeutisch geeigneten Trägerstoffen eine oder mehrere erfindungsgemäße Verbindungen enthalten oder die aus einem oder mehreren erfindungsgemäßen Wirkstoffen bestehen, sowie Verfahren zur Herstellung dieser Zubereitungen.

Zur vorliegenden Erfindung gehören auch pharmazeutische Zubereitungen in Dosierungseinheiten. Dies bedeutet, daß die Zubereitungen in Form einzelner Teile, z.B. Tabletten, Dragees, Kapseln, Pillen, Suppositorien und Ampullen vorliegen, deren Wirkstoffgehalt einem Bruchteil oder einem Vielfachen einer Einzeldosis entspricht. Die Dosierungseinheiten können z.B. 1, 2, 3 oder 4 Einzeldosen oder 1/2, 1/3 oder 1/4 einer Einzeldosis enthalten. Eine Einzeldosis enthält vorzugsweise die Menge Wirkstoff, die bei einer Applikation verabreicht wird und die gewöhnlich einer granzen, einer halben oder einem Drittel oder einem Viertel einer Tragesdosis entspricht,

Unter nicht-toxischen, inerten pharmazeutisch geeigneten Trägerstoffen sind feste, halbfeste oder flüssige Verdünnungsmittel, Füllstoffe und Formulierungshilfsmittel jeder Art zu verstehen.

Als bevorzugte pharmazeutische Zubereitungen seien Tabletten, Dragees, Kapseln, Pillin, Granuate, Suppositorien, Lösungen, Suspension und Emulsionen, Pasten, Salben, Gele, Cremes, Lotions, Puder und Sprays genannt.

Tabletten, Dragees, Kapseln, Pillen und Granulate können den oder die Wirkstoffe neben den üblichen Trägerstoffen enthalten, wie (a) Füll- und Streckmittel, z.B. Stärken, Milchzucker, Rohrzucker, Glukose, Mannit und Kieselsaüre, (b) Bindemittel, z.B. Carboxymethylcellulose, Alginate, Gelatine, Polyvinvylpyrrolidon, (c) Feuchthaltemittel, z.B. Glycerin, (d) Sprengmittel, z.B. Agar-Agar, Calciumcarbonat und Natriumcarbonat, (e) Lösungsverzögerer, z.B. Paraffin und (f) Resorptionsbeschleuniger, z.B. quarternäre Ammoniumverbindungen, (g) Netzmittel, z.B. Cetylalkohol, Glycerinmonostearat, (h) Adsorptionsmittel, z.B. Kaolin und Bentonit und (i) Gleitmittel z.B. Talkum, Calcium- und Magnesiumstearat und feste Polyethylenglykole oder Gemische der unter (a) bis (i) aufegeführten Stoffe.

Die Tabletten, Dragees, Kapseln, Pillen und Granulate können mit den üblichen, gegebenenfalls Opakisierungsmitteln enthaltenden, Überzügen und Hüllen versehen sein und auch so zusammengesetzt sein, daß sie den oder die Wirkstoffe nur oder bevorzugt in einem bestimmten Teil des Intestinaltraktes gegebenenfalls verzögert abgeben, wobei als Einbettungsmassen z.B. Polymersubstanzen und Wachse verwendet werden können.

Der oder die Wirkstoffe können gegebenenfalls mit einem oder mehreren der oben angegebenen Trägerstoffe auch in mikroverkapselter Form vorliegen.

Suppositorien können neben dem oder den Wirkstoffen die üblichen wasserlöslichen oder wasserunlöslichen Trägerstoffe enthalten, z.B. Polyethylenglykole, Fette, z.B. Kakaofett und höhere Ester (z.B. $C_{14}$-Alkohol mit $C_{16}$-Fettsäure) oder Gemische dieser Stoffe.

Salben, Pasten, Cremes und Gele können neben dem oder den Wirkstoffen die üblichen Trägerstoffe enthalten, z.B. tierische und pflanzliche Fette, Wasche, Paraffine, Stärke, Tragant, Cellulosderivate,

Polyethylenglykole, Silikone, Bentonite, Kieselsäure, Talkum und Zinkoxid oder Gemische dieser Stoffe.

Puder und Sprays können neben dem oder den Wirkstoffen die üblichen Trägerstoffe enthalten, z.B. Milchzucker, Talkum, Kieselsäure, Aluminiumhydroxid, Calciumsilikat und Polyamidpulver oder Gemische dieser Stoffe. Sprays können zusätzlich die üblichen Treibmittel, z.B. Chlorfluorkohlenwasserstoffe, enthalten.

Lösungen und Emulsionen können neben dem oder den Wirkstoffen die üblichen Trägerstoffe wie Lösungsmittel, Lösungsvermittler und Emulgatoren, z.B. Wasser, Ethylalkohol, Isopropylalkohol, Ethylcarbonat, Ethylacetat, Benzylalkohol, Benzylbenzoat, Propylenglykol, 1,3-Butylenglykol, Dimethylformamid, Öle, insbesondere Baumwollsaatöl, Erdnußöl, Maiskeimöl, Olivenöl, Ricinusöl und Sesamöl, Glycerin, Glycerinformal, Tetrahydrofurfurylalkohol, Polyethylenglykole und Fettsäureester des Sorbitans oder Gemische dieser Stoffe enthalten.

Zur parenteralen Applikation können die Lösungen und Emulsionen auch in steriler und blutisotonischer Form vorliegen.

Suspensionen können neben dem oder den Wirkstoffen die üblichen Trägerstoffe wie flüssige Verdünnungsmittel, z.B. Wasser, Ethylalkohol, Propylenglykol, Suspendiermittel, z.B. ethoxylierte Isostearylalkohole, Polyoxyethylensorbit- und Sorbitan-Ester, mikrokristalline Cellulose, Aluminiummetahydroxid, Bentonit, Agar-Agar und Tragant oder Gemische dieser Stoffe enthalten.

Die genannten Formulierungsformen können auch Färbemittel, Konservierungsstoffe sowie geruchs- und geschmacksverbesserte Zusätze, z.B. Pfefferminzöl und Eukalyptusöl und Süßmittel, z.B. Saccharin, enthalten.

Die therapeutisch wirksamen Verbindungen sollen in den oben aufgeführten pharmazeutischen Zubereitungen vorzugsweise in einer Konzentration von etwa 0,1 bis 99,5, vorzugsweise von etwa 0,5 bis 95 Gew.-%, der Gesamtmischung vorhanden sein.

Die oben aufgeführten pharmazeutischen Zubereitungen können außer den erfindungsgemäßen Verbindungen auch weitere pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen.

Die genannten Zubereitungen können bei Mensch und Tier entweder oral, rektal, parenteral (intravenös, intramuskulär, subkutan), intracisternal, intravaginal, intraperitoneal, lokal (Puder, Salbe, Tropfen) und zur Therapie von Infektionen in Hohlräumen, Körperhölen angewendet werden. Als geeignete Zubereitungen kommen Injectionlösungen, Lösungen und Suspensionen für die orale Therapie, Gele, Aufgußformulierungen, Emulsionen, Salben oder Tropfen in Frage. Zur lokalen Therapie können ophthalmologische und dermatologische Formulierungen, Silber- und andere Salze, Ohrentropfen, Augensalbe, Pruder oder Lösungen verwendet werden. Bei Tieren kann die Aufnahme auch über das Futter oder Trinkwasser in geeigneten Formulierungen erfolgen. Ferner können Gele, Pulver, Puder, Tabletten, Retard-Tabletten, Premixe, Konzentrate, Granulate, Pellets, Boli, Kapseln, Aerosole, Sprays, Inhalate bei Mensch und Tier angewendet werden. Ferner können die erfindungsgemäßen Verbindungen in andere Trägermaterialien wie zum Beispiel Kunstoffe, (Kunststoffketten zur lokalen Therapie), Kollagen oder Knochenzement eingearbeitet werden.

Im allgemeinen hat es sich sowohl in der Human- als auch in der Veterinärmedizin als vorteilhaft erwiesen, den oder die erfindungsgemäßen Wirkstoffe in Gesamtmengen von etwa 0,5 bis etwa 500, vorzugsweise 5 bis 100 mg/kg Körpergewicht je 24 Stunden, gegebenenfalls in Form mehrerer Eingzelgaben, zur Erzielung der gewünschten Ergebnisse zu verabreichen. Eine Einzelgabe enthält den oder die erfindungsgemäßen Wirkstoffe vorzugsweise in Mengen von etwa 1 bis etwa 80, insbesondere 3 bis 30 mg/kg Körpergewicht. Es kann jedoch erforderlich sein, von den genannten Dosierungen abzuweichen, und zwar in Abhängigkeit von der Art und dem Körpergewicht des zu behandelnden Objekts, der Art und der Schwere der Erkrankung, der Art der Zubereitung und der Applikation des Arzneimittels sowie dem Zeitraum bzw. Intervall, innerhalb welchem die Verabreichung erfolgt.

So kann es in einigen Fällen ausreichend sein, mit weniger als der obengenannten Menge Wirkstoff auszukommen, während in anderen Fällen die oben angeführte Wirkstoffmenge überschritten werden muß. Die Festlegung der jeweils erforderlichen optimalen Dosierung und Applikationsart der Wirkstoffe kann durch jeden Fachmann aufgrund seines Fachwisens leicht erfolgen.

Die neuen Verbindungen können in den üblichen Konzentrationen und Zubereitungen zusammen mit dem Futter bzw. mit Futterzubereitungen oder mit dem Trinkwasser gegeben werden. Dadurch kann eine Infektion durch gram-negative oder gram-positive Bakterien verhindert, gebessert und/oder geheilt werden und dadurch eine Förderung des Wachstums und eine Verbesserung der Verwertung des Futters erreicht werden.

In der nachstehenden Tabelle sind die MHK-Werte einiger der erfindungsgemäßen Verbindungen im Vergleich zu Ciprofloxacin angegeben.

MHK-Werte (mg/l)*

| Keime \ Beispiel | 1 | 3 | Ciprofloxacin |
|---|---|---|---|
| E.coli | | | |
| 4418 | ≤0,015 | ≤0,015 | ≤0,015 |
| Neumann | ≤0,015 | ≤0,015 | ≤0,015 |
| T7 | <0,015 | ≤0,015 | ≤0,015 |
| 455/7 | 32 | 0,25 | 1 |
| A261 | <0,015 | ≤0,015 | ≤0,015 |
| Klebsiella pneum. | | | |
| 63 | ≤0,015 | ≤0,015 | 0,03 |
| 8085 | ≤0,015 | ≤0,015 | ≤0,015 |
| 6179 | 0,125 | ≤0,015 | 0,125 |
| 57USA | 0,125 | ≤0,015 | ≤0,015 |
| 6318 | 0,06 | ≤0,015 | 0,06 |
| Proteus | | | |
| mir.8223 | 2 | 0,06 | 4 |
| 8175 | 0,06 | ≤0,015 | 0,06 |
| vulg.1017 | ≤0,015 | ≤0,015 | ≤0,015 |
| morg.932 | ≤0,015 | ≤0,015 | ≤0,015 |
| 11006 | ≤0,015 | ≤0,015 | ≤0,015 |
| Providencia stuartei | | | |
| 12012 | ≤0,015 | ≤0,015 | ≤0,015 |
| 12052 | 32 | 1 | 16 |
| Serratia marc. | | | |
| 16040 | 32 | 1 | 8 |
| Staph. aureus | | | |
| FK422 | ≤0,015 | ≤0,015 | 0,5 |
| 1756 | ≤0,015 | ≤0,015 | 0,25 |
| 133 | ≤0,015 | ≤0,015 | 0,25 |
| Strepto. faecalis | | | |
| 27101 | 0,125 | ≤0,015 | 0,25 |
| 9790 | 0,125 | ≤0,015 | 0,5 |
| Psdm. aeruginosa | | | |
| Walter | 1 | 0,25 | 0,5 |
| Ellsworth | 0,125 | ≤0,015 | 0,06 |

*) Agardilutionstest (Multipoint-Inokulator)
Isosensitestmedium pH 7,2

Beispiel 1

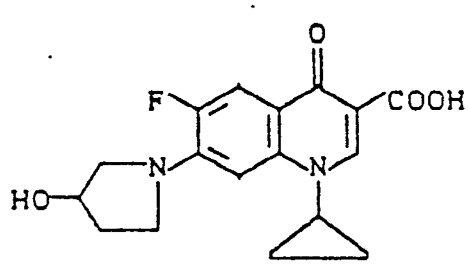

7,95 g (30 mmol) 1-Cyclopropyl-6,7-difluor-1,4-dihydro-4-oxo-3-chinolincarbonsäure werden in einer Mischung aus 60 ml Acetonitril und 30 ml Dimethylformamid mit 3,9 g (31 mmol) 3-Hydroxypyrrolidin-Hydrochlorid (Racemat) und 9,9 g (88 mmol) 1,4-Diazabicyclo[2.2.2]octan versetzt und 3 Stunden unter Rückfluß erhitzt. Die Suspension wird eingeengt, der Rückstand mit etwa 100 ml Wasser verrührt und mit 2 n-Salzsäure auf pH 6—7 eingestellt. Das ungelöste Reaktionsprodukt wird abgesaugt, mit Wasser gewaschen und getrocknet.

Ausbeute: 9,3 g (93,3% der Theorie) 1-Cyclopropyl-6-fluor-1,4-dihydro-7-(3-hydroxy-1-pyrrolidinyl)-4-oxo-3-chinolincarbonsäure vom Schmelzpunkt 325°C—328°C (unter Zersetzung).

Beispiel 2

2,65 g (10 mmol) 1-Cyclopropyl-6,7-difluor-1,4-dihydro-4-oxo-3-chinolincarbonsäure werden in 6 ml Dimethylsulfoxid mit 1 g (11 mmol) 3-Hydroxypyrrolidin (Racemat) und 2,2 g (20 mmol) 1,4-Diazabicyclo[2.2.2]octan 3 Stunden auf 130°C erhitzt. Die Suspension wird nach dem Abkühlen mit 30 ml Wasser verrührt, mit 2 n-Salzsäure auf pH 6—7 eingestellt, der Niederschlag abgesaugt, mit Wasser gewaschen und in 30 ml Glykolmonomethylether aufgekocht. Man erhält 2,7 g (81% der Theorie) 1-Cyclopropyl-6-fluor-1,4-dihydro-7-(3-hydroxy-1-pyrrolidinyl)-4-oxo-3-chinolincarbonsäure vom Schmelzpunkt 332—336°C (unter Zersetzung). Die Verbindung ist dünnschichtchromatographisch identisch mit der Verbindung aus Beispiel 1.

Beispiel 3

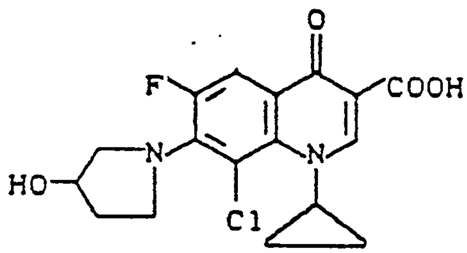

1,5 g (5 mmol) 8-Chlor-1-cyclopropyl-6,7-difluor-1,4-dihydro-4-oxo-3-chinolincarbonsäure werden in 10 ml Acetonitril und 5 ml Dimethylformamid mit 1,1 g (10 mmol) 1,4-Diazabicyclo[2.2.2]octan und 650 mg (5,3 mmol) 3-Hydroxypyrrolidin-Hydrochlorid versetzt und 3 Stunden unter Rückfluß erhitzt. Die Suspension wird eingeengt, der Rückstand mit Wasser verrührt, mit 2 n-Salzsäure auf pH 6—7 eingestellt, der Niederschlag abgesaugt, getrocknet und aus Glykolmonomethylether umkristallisiert.

Ausbeute: 1,2 g (65% der Theorie) 8-Chlor-1-cyclopropyl-7-fluor-1,4-dihydro-7-(3-hydroxy-1-pyrrolidinyl)-4-oxo-3-chinolincarbonsäure vom Schmelzpunkt 221—223°C (unter Zersetzung).

Beispiel 4

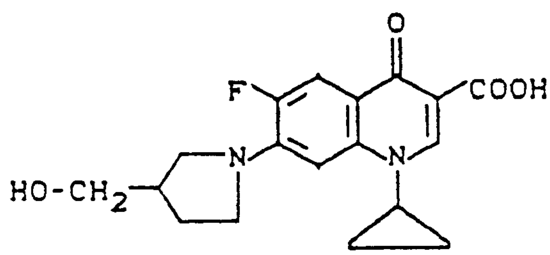

Man setzt analog Beispiel 1 mit 3-Hydroxymethyl-pyrrolidin um und erhält 1-Cyclopropyl-6-fluor-1,4-dihydro-7-(3-hydroxymethyl-1-pyrrolidinyl)-3-chinolincarbonsäure vom Schmelzpunkt 278—281°C (unter Zersetzung).

Analog erhält man:

8-Chlor-1-cyclopropyl-6-fluor-1,4-dihydro-7-(3-hydroxymethyl-1-pyrrolidinyl)-3-chinolincarbonsäure vom Schmelzpunkt 160—162° (unter Zersetzung);

1-Cyclopropyl-6,8-difluor-1,4-dihydro-7-(3-hydroxymethyl-1-pyrrolidinyl)-3-chinolincarbonsäure vom Schmelzpunkt 236—240°C (unter Zersetzung).

**Patentansprüche**

1. 7-(1-Pyrrolidinyl)-3-chinoloncarbonsäure-Derivate der Formel (I)

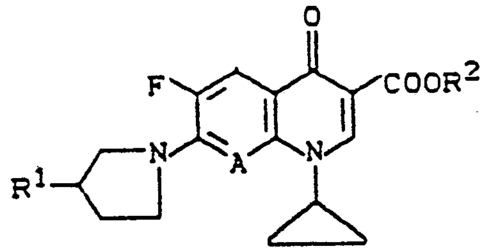

(I),

in welcher
A für CH, CCl oder CF
$R^1$ für Hydroxy oder Hydroxymethyl und
$R^2$ für Wasserstoff, Methyl oder Ethyl steht, mit der Maßgabe, daß für den Fall, daß $R^1$ für Hydroxy steht, A nicht für den Rest CF stehen kann, und deren pharmazeutisch verwendbaren Hydrate sowie die Alkali-, Erdalkali-, Silber- und Guanidiniumsalze der zugrundeliegenden Carbonsäuren.

2. 7-(1-Pyrrolidinyl)-3-chinoloncarbonsäure-Derivate der Formel (I)

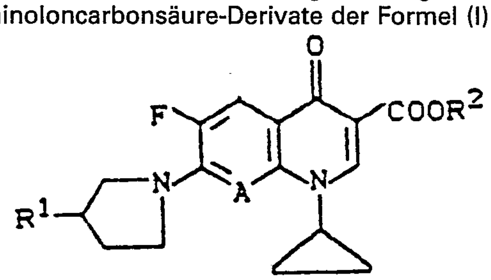

(I),

in welcher
A für CH, CCl oder CF
$R^1$ für Hydroxy oder Hydroxymethyl und
$R^2$ für Wasserstoff, Methyl oder Ethyl steht, mit der Maßgabe, daß für den Fall, daß $R^1$ für Hydroxy steht, A nicht für den Rest CF stehen kann, und deren pharmazeutisch verwendbaren Hydrate sowie die Alkali-, Erdalkali-, Silber- und Guanidiniumsalze der zugrundeliegenden Carbonsäuren zur Verwendung in einem Verfahren zur therapeutischen Behandlung des menschlichen oder tierischen Körpers.

3. Verfahren zur Herstellung von 7-(1-Pyrrolidinyl)-3-chinoloncarbonsäure-Derivaten der Formel (I)

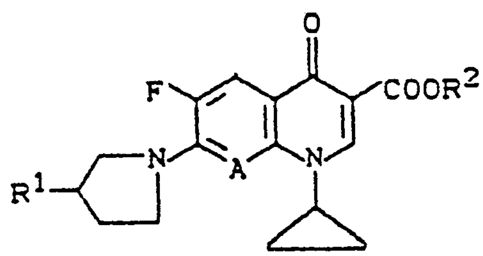

(I),

in welcher
A für CH, CCl oder CF
$R^1$ für Hydroxy oder Hydroxymethyl und
$R^2$ für Wasserstoff, Methyl oder Ethyl steht, mit der Maßgabe, daß für den Fall, daß $R^1$ für Hydroxy steht, A nicht für den Rest CF stehen kann, und deren pharmazeutisch verwendbaren Hydraten sowie deren Alkali-, Erdalkali-, Silber- und Guanidiniumsalze der zugrundeliegenden Carbonsäuren, dadurch gekennzeichnet, daß man Verbindungen der Formel (II)

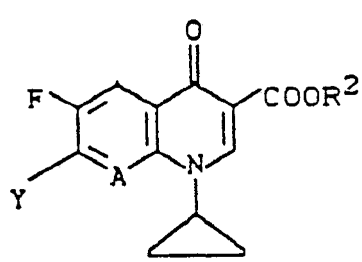

(II),

in welcher
A und $R^2$ die oben angegebene Bedeutung haben und
Y für Halogen, insbesondere Fluor oder Chlor, steht, mit Pyrrolidinen der Formel (III)

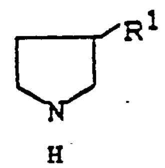

(III),

in welcher $R^1$ die oben angegebene Bedeutung hat, gegebenenfalls in Gegenwart von Säurebindungsmitteln umsetzt.

4. Verfahren zur Herstellung von Verbindungen der Formel (I)

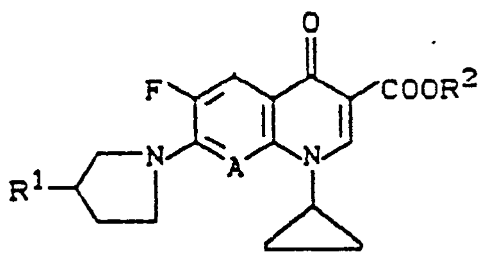

(I),

in welcher
A und $R^1$ die in Anspruch 1 angegebene Bedeutung haben und
$R^2$ die in Anspruch 3 angegebene Bedeutung hat, aber nicht Wasserstoff sein kann, dadurch gekennzeichnet, daß man eine Verbindung (IV)

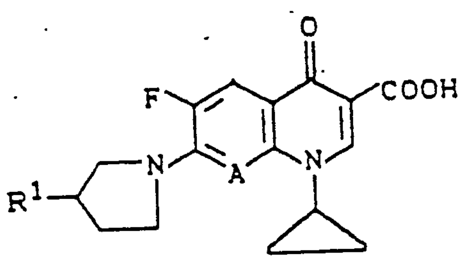

(IV),

in welcher $R^1$ und A die oben angegebene Bedeutung haben, mit Verbindungen der Formel (V)

$$R^2\text{—}Z \quad (V),$$

in welcher $R^2$ die oben angegebene Bedeutung hat, aber nicht Wasserstoff sein kann, und
Z für Hydroxy oder Halogen, insbesondere Chlor, Brom oder Iod, steht, gegebenenfalls in Gegenwart von Säurebindungsmitteln oder unter wasserabspaltenden Bedingungen umsetzt.

5. Arzneimittel, enthaltend 7-(1-Pyrrolidinyl)-3-chinoloncarbonsäure-Derivate der Formel (I)

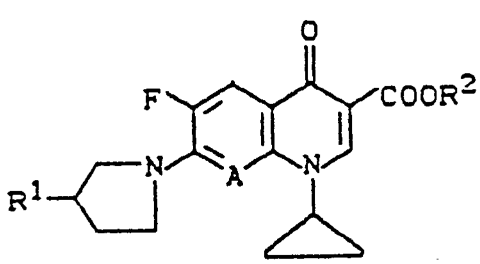

(I),

in welcher
A für CH, CCl oder CF
$R^1$ für Hydroxy oder Hydroxymethyl und
$R^2$ für Wasserstoff, Methyl oder Ethyl steht, mit der Maßgabe, daß für den Fall, daß $R^1$ für Hydroxy steht, A nicht für den Rest CF stehen kann, und deren pharmazeutisch verwendbaren Hydrate sowie die

Alkali-, Erdalkali-, Silber- und Guanidiniumsalze der zugrundeliegenden Carbonsäuren.

6. Verwendung von 7-(1-Pyrrolidinyl)-3-chinoloncarbonsäure-Derivaten der Formel (I)

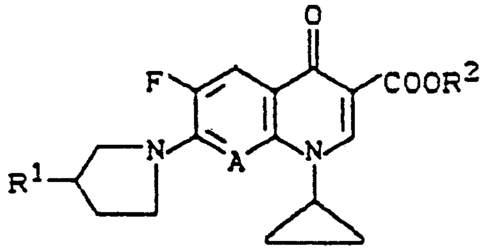

(I),

in welcher

A für CH, CCl oder CF

$R^1$ für Hydroxy oder Hydroxymethyl und

$R^2$ für Wasserstoff, Methyl oder Ethyl steht, mit der Maßgabe, daß für den Fall, daß $R^1$ für Hydroxy steht, A nicht für den Rest CF stehen kann, und deren pharmazeutisch verwendbaren Hydraten sowie die Alkali-, Erdalkali-, Silber- und Guanidiniumsalze der zugrundeliegenden Carbonsäuren, zur Herstellung von Arzneimetteln.

7. Tierarzneimittel, enthaltend 7-(1-Pyrrolidinyl)-3-chinoloncarbonsäure-Derivate der Formel (I)

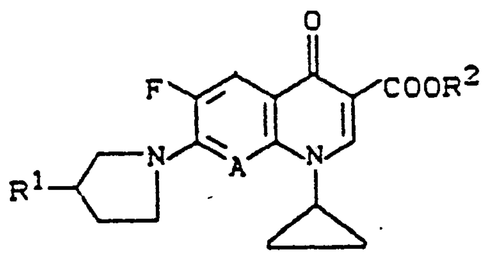

(I),

in welcher

A für CH, CCl oder CF

$R^1$ für Hydroxy oder Hydroxymethyl und

$R^2$ für Wasserstoff, Methyl oder Ethyl steht, mit der Maßgabe, daß für den Fall, daß $R^1$ für Hydroxy steht, A nicht für den Rest CF stehen kann, und deren pharmazeutisch verwendbaren Hydrate sowie die Alkali-, Erdalkali-, Silber- und Guanidiniumsalze der zugrundeliegenden Carbonsäuren.

8. Tierfutterzusätze, enthaltend 7-(1-Pyrrolidinyl)-3-chinoloncarbonsäure-Derivate der Formel (I)

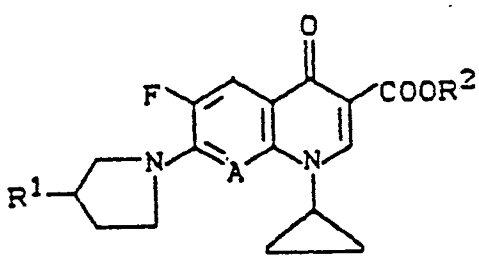

(I),

in welcher

A für CH, CCl oder CF

$R^1$ für Hydroxy oder Hydroxymethyl und

$R^2$ für Wasserstoff, Methyl oder Ethyl steht, mit der Maßgabe, daß für den Fall, daß $R^1$ für Hydroxy steht, A nicht für den Rest CF stehen kann, und deren pharmazeutisch verwendbaren Hydrate sowie die Alkali-, Erdalkali-, Silber- und Guanidiniumsalze der zugrundeliegenden Carbonsäuren.

**Revendications**

1. Dérivés de l'acide 7-(1-pyrrolidinyl)-3-quinolonecarboxylique, de formule (I)

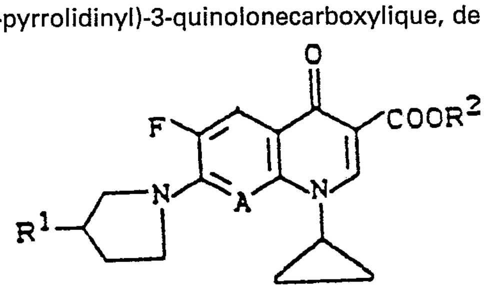

(I),

(dans laquelle
A représente CH, CCl, ou CF,
$R^1$ représente un groupe hydroxyle ou hydroxyméthyle, et
$R^2$ représente un atome d'hydrogène ou un groupe méthyle ou éthyle, à la condition qu'au cas où $R^1$ représente un groupe hydroxyle, A ne puisse représenter le reste CF), et leurs hydrates pharmaceutiquement applicables ainsi que les sels alcalins, alcalino-terreux, d'argent et de guanidinium, des acides carboxyliques se trouvant à la base de ces dérivés.

2. Dérivés de l'acide 7-(1-pyrrolidinyl)-3-quinolonecarboxylique, de formule (I)

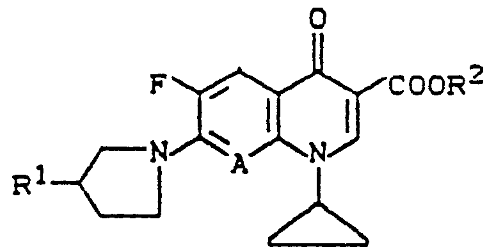

(I),

(dans laquelle
A représente CH, CCl, ou CF,
$R^1$ représente un groupe hydroxyle ou hydroxyméthyle, et
$R^2$ représente un atome d'hydrogène, un groupe méthyle ou éthyle, à la condition qu'au cas où $R^1$ représente un groupe hydroxyle, A ne puis se représenter le reste CF), et leurs hydrates utilisables pharmaceutiquement ainsi que les sels alcalins, alcalino-terreux, d'argent et de guanidinium, des acides carboxyliques se trouvant à la base de ces dérivés, à utiliser dans un procédé de traitement thérapeutique du corps humain ou animal.

3. Procédé pour préparer des dérives de l'acide 7-(1-pyrrolidinyl)-3-quinolonecarboxylique, de formule (I)

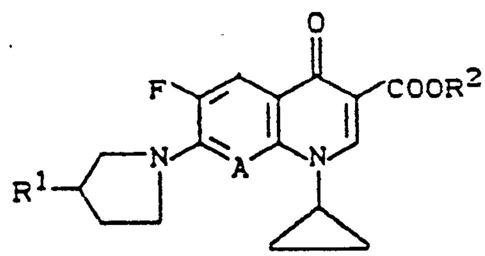

(I),

(dans laquelle
A représente CH, CCl, ou CF,
$R^1$ représente un groupe hydroxyle ou hydroxyméthyle, et
$R^2$ représente un atome d'hydrogène ou un groupe méthyle ou éthyle, à la condition qu'au cas où $R^1$ représente un groupe hydroxyle, A ne puisse représenter le reste CF), et leurs hydrates pharmaceutiquement utilisables ainsi que des sels alcalins, alcalino-terreux, d'argent et de guanidinium, des acides carboxyliques se trouvant à la base de ces dérivés, procédé caractérisé en ce qu'on fait réagir des composés de formule (II)

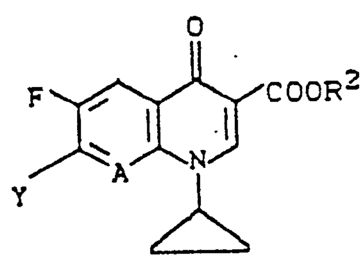

(II),

(dans laquelle
A et $R^2$ ont le sens précité, et
Y représente un atome d'halogène, en particulier le flour ou le chlore) avec des pyrrolidines de formule (III)

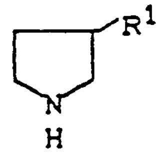

(III),

(dans laquelle $R^1$ a le sens précité), en opérant éventuellement en présence d'agents de fixation des acides.

4. Procédé pour préparer des composés de formule (I)

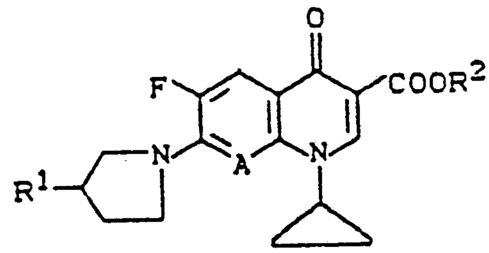

(I),

(dans laquelle

A et $R^1$ ont le sens indiqué à la revendication 1, et

$R^2$ a le sens indiqué à la revendication 3, mais ne peut être un atome d'hydrogène), procédé caractérisé en ce qu'on fait réagir un composé (IV)

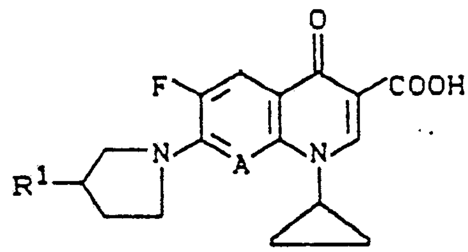

(IV),

(dans laquelle $R^1$ et A ont le sens indiqué ci-dessus) avec des composés de formule (V)

$$R^2—Z \qquad (V)$$

(dans laquelle $R^2$ a le sens précité, mais ne peut représenter un atome d'hydrogène, et

Z représente un groupe hydroxyl ou un atome d'halogène, en particulier le chlore, le brome ou l'iode), en opérant éventuellement en présence d'agents de fixation des acides ou dans des conditions provoquant la séparation de l'eau.

5. Médicament contenant des dérivés de l'acide 7-(1-pyrrolidinyl)-3-quinolone-carboxylique, de formule (I)

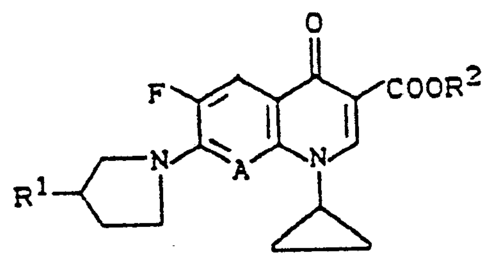

(I),

(dans laquelle

A représente CH, CCl, ou CF,

$R^1$ représente un groupe hydroxyle ou hydroxyméthyle, et

$R^2$ représente un atome d'hydrogène ou un groupe méthyle ou éthyle, à la condition qu'au cas où $R^1$ représente un groupe hydroxyle, A ne puisse représenter le reste CF), et leurs hydrates pharmaceutiquement utilisables, ainsi que les sels alcalins, alcalino-terreux, d'argent et de guanidinium, des acides carboxyliques se trouvant à la base de ces dérivés.

6. Utilisation de dérivés de l'acide 7-(1-pyrrolidinyl)-3-quinolone-carboxylique, de formule (I)

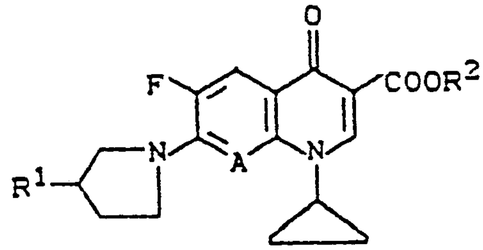

(I),

(dans laquelle

A représente CH, CCl, ou CF,

$R^1$ représente un groupe hydroxyle ou hydroxyméthyle, et

$R^2$ représente un atome d'hydrogène ou un groupe méthyle ou éthyle, à la condition qu'au cas où $R^1$ représente un groupe hydroxyle, A ne puisse représenter le reste CF), et de leurs hydrates pharmaceutiquement utilisables, ainsi que des sels alcalins, alcalino-terreux, d'argent et de guanidinium, des acides carboxyliques se trouvant à la base de ces dérivés, pour préparer des médicaments.

7. Médicament pour animaux, ce médicament contenant des dérivés d'un acide 7-(1-pyrrolidinyl)-3-quinolone-carboxylique, de formule (I)

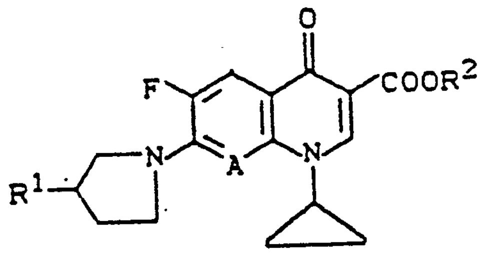

(I),

(dans laquelle

A représente CH, CCl, ou CF,

$R^1$ représente un groupe hydroxyle ou hydroxyméthyle, et

$R^2$ représente un atome d'hydrogène ou un groupe méthyle ou éthyle, à la condition qu'au cas où $R^1$ représente un groupe hydroxyle, A ne puisse représenter le reste CF), et leurs hydrates pharmaceutiquement utilisables, ainsi que les sels alcalins, alcalino-terreux, d'argent et de guanidinium, des acides carboxyliques se trouvant à la base de ces dérivés.

8. Additif pour l'alimentation des animaux, ces additifs contenant des dérivés d'un acide 7-(1-pyrrolidinyl)-3-quinolonecarboxylique, de formule (I)

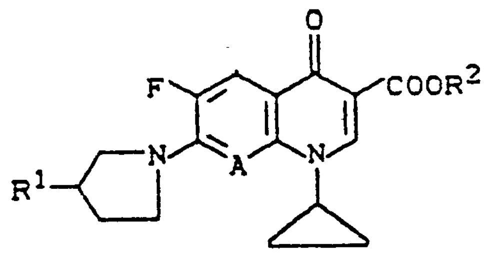

(I),

(dans laquelle

A représente CH, CCl, ou CF,

$R^1$ représente un groupe hydroxyle ou hydroxyméthyle, et

$R^2$ représente un atome d'hydrogène ou un groupe méthyle ou éthyle, à la condition qu'au cas où $R^1$ représente un groupe hydroxyle, A ne puisse représenter le reste CF), et leurs dérivés pharmaceutiquement utilisables, ainsi que les sels alcalins, alcalino-terreux, d'argent et de guanidinium, des acides carboxyliques se trouvant à la base de ces dérivés.

**Claims**

1. 7-(1-Pyrrolidinyl)-3-quinolonecarboxylic acid derivatives of the formula (I)

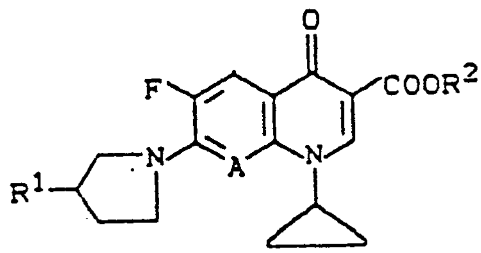

(I),

in which

A stands for CH, CCl or CF,

$R^1$ stands for hydroxyl or hydroxymethyl and

$R^2$ stands for hydrogen, methyl or ethyl, with the proviso that in the case where $R^1$ stands for hydroxyl

A cannot stand for the radical CF, and their pharmaceutically usable hydrates and also the alkali metal, alkaline earth metal, silver and guanidinium salts of the parent carboxylic acids.

2. 7-(1-Pyrrolidinyl)-3-quinolonecarboxylic acid derivatives of the formula (I)

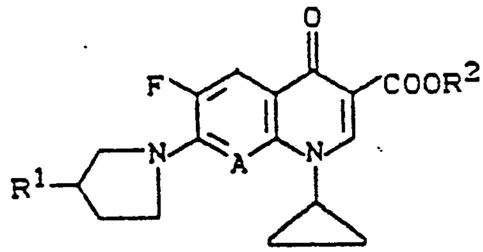

(I),

in which

A stands for CH, CCl or CF,

$R^1$ stands for hydroxyl or hydroxymethyl and

$R^2$ stands for hydrogen, methyl or ethyl, with the proviso that in the case where $R^1$ stands for hydroxyl A cannot stand for the radical CF, and their pharmaceutically usable hydrates and also the alkali metal, alkaline earth metal, silver and guanidinium salts of the parent carboxylic acids for use in a method for the therapeutic treatment of the human or animal body.

3. Process for the preparing 7-(1-pyrrolidinyl)-3-quinolonecarboxylic acid derivatives of the formula (I)

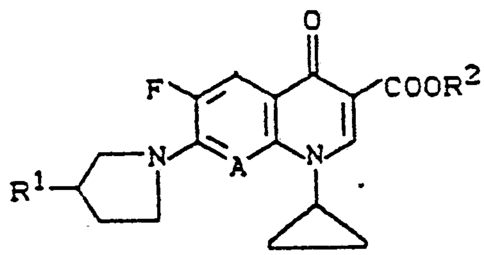

(I),

in which

A stands for CH, CCl or CF,

$R^1$ stands for hydroxyl or hydroxymethyl and

$R^2$ stands for hydrogen, methyl or ethyl, with the proviso that in the case where $R^1$ stands for hydroxyl A cannot stand for the radical CF, and their pharmaceutically usable hydrates and also their alkali metal. alkaline earth metal, silver and guanidinium salts of the parent carboxylic acids, characterized in that compounds of the formula (II)

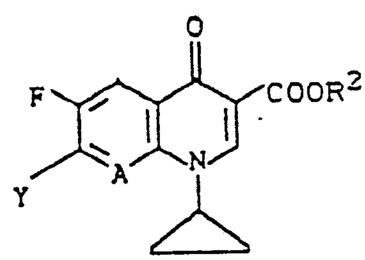

(II),

in which

A and $R^2$ have the abovementioned meaning and

Y stands for halogen, in particular fluorine of chlorine, are reacted with pyrrolidines of the formula (III)

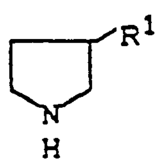

(III),

in which

$R^1$ has the abovementioned meaning, if desired in the presence of acid-binding agents.

4. Process for preparing compounds of the formula (I)

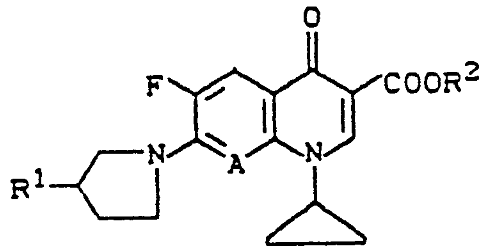

(I),

in which

A and $R^1$ have the meaning mentioned in Claim 1 and

$R^2$ has the meaning mentioned in Claim 3 but cannot be hydrogen, characterized in that a compound (IV)

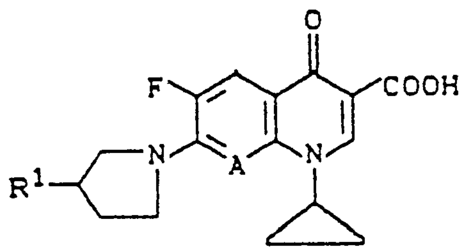

(IV),

in which

$R^1$ and A have the abovementioned meaning is reacted with compounds of the formula (V)

$$R^2—Z \qquad (V)$$

in which

$R^2$ has the abovementioned meaning but cannot be hydrogen, and

Z stands for hydroxyl or halogen, in particular chlorine, bromine or iodine, if desired in the presence of acid-binding agents or under water-eliminating conditions.

5. Medicament containing 7-(1-pyrrolidinyl)-3-quinolonecarboxylic acid derivatives of the formula (I)

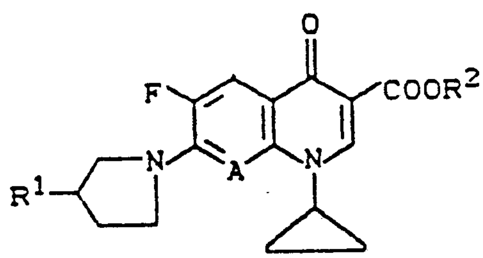

(I),

in which

A stands for CH, CCl or CF,

$R^1$ stands for hydroxyl or hydroxymethyl and

$R^2$ stands for hydrogen, methyl or ethyl, with the proviso that in the case where $R^1$ stands for hydroxyl A cannot stand for the radical CF, and their pharmaceutically usable hydrates and also the alkali metal, alkaline earth metal, silver and guanidinium salts of the parent carboxylic acids.

6. Use of 7-(1-pyrrolidinyl)-3-quinolonecarboxylic acid derivatives of the formula (I)

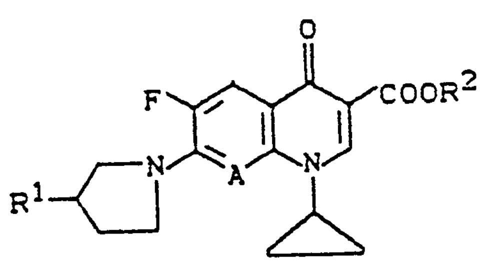

(I),

in which

A stands for CH, CCl or CF,

$R^1$ stands for hydroxyl or hydroxymethyl and

$R^2$ stands for hydrogen, methyl or ethyl, with the proviso that in the case where $R^1$ stands for hydroxyl A cannot stand for the radical CF, and their pharmaceutically usable hydrates and also the alkali metal, alkaline earth metal, silver and guanidinium salts of the parent carboxylic acids, for preparing medicaments.

7. Veterinary medicament containing 7-(1-pyrrolidinyl)-3-quinolonecarboxylic acid derivatives of the formula (I)

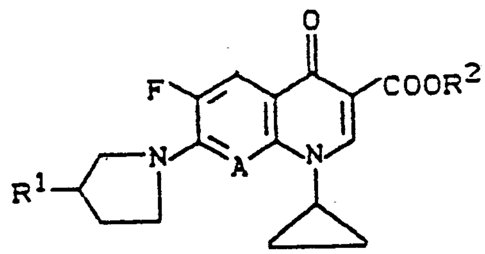

(I),

in which

A stands for CH, CCl or CF,

$R^1$ stands for hydroxyl or hydroxymethyl and

$R^2$ stands for hydrogen, methyl or ethyl, with the proviso that in the case where $R^1$ stands for hydroxyl A cannot stand for the radical CF, and their pharmaceutically usable hydrates and also the alkali metal, alkaline earth metal, silver and guanidinium salts of the parent carboxylic acids.

8. Animal fodder additives containing 7-(1-pyrrolidinyl)-3-quinolonecarboxylic acid derivatives of the formula (I)

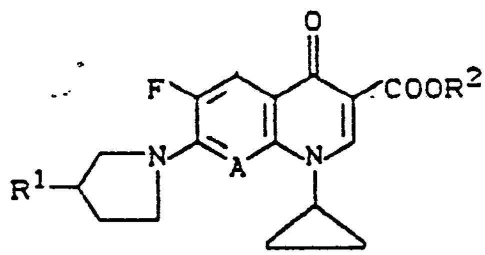

(I),

in which

A stands for CH, CCl or CF,

$R^1$ stands for hydroxyl or hydroxymethyl and

$R^2$ stands for hydrogen, methyl or ethyl, with the proviso that in the case where $R^1$ stands for hydroxyl A cannot stand for the radical CF, and their pharmaceutically usable hydrates and also the alkali metal, alkaline earth metal, silver and guanidinium salts of the parent carboxylic acids.